# EUROPEAN PATENT APPLICATION

(11) **EP 4 736 863 A1**
(43) Date of publication of application: **06.05.2026**
(21) Application number: 24830791.0
(22) Date of filing: 26.06.2024
(51) Int. Cl.: A61K 36/61, A61P 11/04, A61P 11/02, A61K 31/045, A61K 9/12

(54) **NOVEL MENTHOL-EUCALYPTUS OIL ANTITUSSIVE SPRAY AND USE THEREOF**

(30) Priority: 30.06.2023 CN 202310803538
(71) Applicant: Sino-Shine Biopharmaceutical Co., Ltd, Hangzhou, Zhejiang 310053 (CN)
(72) Inventor: LI, Guanhan, Hangzhou, Zhejiang 310053 (CN)
(74) Representative: Renaudo, Adrien Hanouar
(86) International application number: PCT/CN2024/101507
(87) International publication number: WO 2025/002152

(57) **Abstract**

Provided is a novel menthol-eucalyptus oil antitussive spray and use thereof in preparation of a drug for treating coughs. The novel menthol-eucalyptus oil antitussive spray includes two natural botanicals, namely, menthol and eucalyptus oil, as active ingredients that work synergistically, and does not contain alcohol. The novel menthol-eucalyptus oil antitussive spray is directly administered to a respiratory tract in a form of spray, and has advantages of low dosage, rapid pharmaceutical action, and great safety.

## Description

The present application claims priority to Chinese Patent Application No. CN202310803538.5 filed to the China National Intellectual Property Administration (CNIPA) on June 30, 2023, and entitled "NOVEL MENTHOL-EUCALYPTUS OIL ANTITUSSIVE SPRAY AND USE THEREOF", which is incorporated herein by reference in its entirety.

### TECHNICAL FIELD

The present disclosure pertains to the fields of pharmaceutics and therapeutics for respiratory diseases, specifically providing a novel menthol-eucalyptus oil antitussive spray and its use thereof.

### BACKGROUND

Menthol, also known as L-1-methyl-4-isopropylcyclohexan-3-ol, is produced or extracted from the fresh stems and leaves of Lamiaceae plant *Mentha haplocalyx* Briq through distillation, lyophilization, and recrystallization.

Menthol is widely used in pharmaceuticals, health, food, cosmetic and other fields. For example, menthol can be used as a flavoring agent in beverages, candies, toothpastes, perfumes, foods, etc. In medicine, menthol is usually used for cooling, relieving itches and pain on the skin or mucous membrane, treating headache, neuralgia, or inflammation from nose, pharynx, and throat. It can also be taken orally as a carminative in traditional Chinese medicine.

In pharmacology, menthol has effects such as cooling, itch relief, analgesia, antisepsis, and anesthesia, and shows modest antibacterial and antiviral effects. The cooling effect of menthol is triggered through a cold sensing receptor, transient receptor potential melastatin (TRPM) 8^{[1]}, a member of the transient receptor potential (TRP) channels family. Activation of TRPM8 by cooling compounds, such as menthol, leads to membrane potential depolarization and Ca²⁺ ion influx, further opening the signaling channel. TRPM8 is expressed in the cold-responsive sensory neurons of dorsal root ganglia and trigeminal nerve. Menthol and cold stimulation can be conducted via the afferent vagus nerve of the lung, bronchi and trachea. Expression levels of TRPM8 vary in the mesenteric vagal ganglia, stomach, vascular smooth muscle, liver, and bladder epithelium^{[1, 2]}. Another TRP family member, TRPV1, is a capsaicin receptor mainly expressed in sensory nerves. TRPV1-positive nerves innervate the entire respiratory tract, including the upper respiratory tract (nose, larynx, and trachea) and lung parenchyma (alveoli, smooth muscle, and blood vessels). TRPV1 agonists, such as capsaicin and citrate, can evoke coughs in humans and guinea pigs. The animal experiments of the present disclosure and other studies show that capsaicin or citric acid-evoked coughs can be significantly suppressed by menthol^{[3, 4]}. Although the mechanism of antitussive effect by menthol is not exactly known, it is probably achieved by elevating the threshold of capsaicin or citric acid-inducing coughs. The antitussive effect of menthol has also been well verified in human clinical trials (including adults and children)^{[5-9]}. Opposite to antitussive effect, menthol can stimulate swallowing, thereby eliminating the concerns regarding an anesthetic effect on the laryngopharynx. In addition, studies also shows that menthol has an expectorant effect.

The antitussive effect of menthol is not recorded in the Chinese Pharmacopoeia, but it is used in a variety of antitussive preparations of traditional Chinese medicine as an assistant component or oral hygiene formulations. In United States, menthol is used in over-the-counter cough drops (e.g., Halls Cough Drops, Ricola Natural Herb Cough Drops, Honees Cough Drops, and CVS Cough Drops) and syrups (e.g., NatraBio Adult Cough Syrup).

Eucalyptus oil, a volatile oil, is made by distillation from *Eucalyptus globulus* Labill in the Myrtaceae family, *Cinnamomum camphora* (L.) Sieb in the Lauraceae family, or other plants of these two families. According to the Chinese Pharmacopoeia, eucalyptus oil is required to contain not less than 70.0% (g/g) of eucalyptol or 1,8-cineol.

In traditional Chinese medicine, eucalyptus oil is believed to possess the effect of dispelling chill and relieving pain, and is used to treat itches and neuralgia. In Western countries, eucalyptus oil is also used for treating upper respiratory tract infections, coughs, bronchitis and other conditions. Eucalyptol, a major component of the eucalyptus oil, has been proven to have anti-inflammatory and antitussive effects in clinical trials^{[10-12]}.

### DETAILED DESCRIPTION OF THE INVENTION

The present disclosure provides a novel antitussive spray based on menthol and eucalyptus oil, featuring an additive or synergistical antitussive effect by incorporating two drugs in one preparation. It exerts a rapid action to directly suppress coughs through a non-traditional, topical administration on the mucosa of respiratory tract. Currently, there are no actual cough suppressant sprays on the market. Some so-called "cough suppressant sprays" available online are essentially traditional Chinese medicine preparations exclusively for treating pharyngitis, without indication for cough treatment included in their instructions. Furthermore, no aerosol spray of antitussive drug is included in the 2020 edition of the Chinese Pharmacopoeia, and no antitussive products have been approved for topical use by the China Food and Drug Administration (CFDA) and shown on the market so far.

The antitussive mechanisms and corresponding receptors are relatively clear for menthol (refer to the BACKGROUND section). In animal experiments, menthol can effectively suppress coughs evoked by both capsaicin and citric acid. However, the mechanism for eucalyptus oil remains unclear, although the antitussive effect of its main ingredient, eucalyptol, has been confirmed in clinical trials^{[10-12]}. Eucalyptus oil has no significant antitussive effect in the capsaicin-guinea pig model^{[3]}, but both menthol and eucalyptus oil show antitussive effects in the citrate-guinea pig model. The effects of both drugs were further verified in the researches in the present disclosure (refer to Animal Experiment 1). In the citrate model, the cough suppression rate was 37.39% and 33.88% respectively while 2% menthol or 3.6% eucalyptus oil was used. The rate was 68.11% when two of both (2% menthol plus 3.6% eucalyptus oil) were co-administered, indicating that the combination leads to a significantly increased antitussive effect (refer to Animal Experiment 1). In this model, the concentration ratio of menthol and eucalyptus oil is approximately 1:2 (2% vs 3.6%) to achieve a similar level of antitussive effect (37.39% vs 33.88%) for the single use of each.

Animal models and clinical trials have confirmed that eucalyptol is an effective anti-inflammatory agent^{[12-14]}. Its anti-inflammatory effect is mainly achieved by suppressing the production of inflammatory factors, such as leukotriene B4 (LTB4), prostaglandin (PGE2), and a variety of cytokines (TNF-α, IL-1β, IL-4, IL-5, IL-6, IL-8, and IL-10). In clinical trials for asthma, eucalyptol can partially replace the use of glucocorticoids. Eucalyptus oil also shows fair antibacterial activities^{[15, 16]}. Therefore, it is possible for eucalyptus oil to exert an antitussive effect through anti-inflammatory effects. However, current animal models are not specifically utilized to test the antitussive effect of drugs on inflammatory coughs. The present disclosure developed a new antitussive animal model for studying inflammatory coughs for the first time. In this model, a pretreatment step of LPS was added to the traditional citrate-guinea pig model to evoke inflammatory coughs (refer to Animal Experiment 2). The LPS pretreatment significantly increased the sensitivity of cough induction to sodium citrate in guinea pigs and an equivalent frequency of coughs was attained using only half the dose of sodium citrate in the original model.

In the novel model, the rate of cough suppression was 39.78% and 36.47% respectively when 4% menthol or 0.15% eucalyptus oil was administered (refer to Animal Experiment 2). Accordingly, the concentration ratio of menthol and eucalyptus oil was over 20:1 for generating similar levels of antitussive effect. Furthermore, it was observed that the antitussive sensitivity of menthol on inflammatory coughs declined, while the effect of eucalyptus oil significantly increased. Based on the results from the original model described above, 2% menthol generated a cough suppression rate of 37.39%. Compared with 4% menthol achieving a similar cough suppression rate in the novel model (refer to Animal Experiment 1, Table 2; Animal Experiment 2, Tables 4 and 6), the antitussive effect of menthol on inflammatory coughs decreased by twofold. Since the concentration of sodium citrate in the novel model was 0.2 M, only half of the concentration in the original model, thus the actual antitussive effect of menthol on inflammatory coughs should be decreased by about four times. In contrast, eucalyptus oil reached a cough suppression rate of 33.88% at a concentration of 3.6% in the original model but gained a suppression rate of 36.47% at a concentration of 0.15% in the novel inflammatory cough model (refer to Animal Experiment 1, Table 2; Animal Experiment 2, Tables 4 and 6), indicating that the antitussive efficacy increased by 24 times. For the same reason, the concentration of sodium citrate for evoking an equivalent number of coughs in the novel model was only half of the value in the original model, so the actual antitussive efficacy of eucalyptus oil on inflammatory coughs was at least 12 times better than on non-inflammatory coughs. Such a great improvement of antitussive effect was totally unexpected for eucalyptus oil on inflammatory coughs. In comparison, for achieving a cough suppression rate of 68.11% by the combination of 2% menthol and 3.6% eucalyptus oil in the traditional model, a suppression rate of 67.57% could be accomplished with only 1% menthol and 0.15% eucalyptus oil co-administrated in the novel informatory cough model (refer to Animal Experiment 2, Tables 4 and 6). This suggests that the dosage of the aerosol menthol-eucalyptus oil preparation can be significantly reduced when applied for inflammatory coughs.

Again, the animal experiments of the disclosure indicate that the antitussive mechanisms of menthol and eucalyptus oil are different. Menthol is better than eucalyptus oil for non-inflammatory coughs, but eucalyptus oil is significantly better than menthol for inflammatory coughs. In other words, menthol is more suitable for the treatment of subacute, chronic, refractory, or stubborn coughs, while eucalyptus oil has a particular advantage for treating coughs resulted from acute upper respiratory tract infections, bronchitis, and pneumonia. The preparations composed of menthol and eucalyptus oil can complement each other's advantages and achieve a synergistic effect in the antitussive mechanisms of both drugs. This will markedly expand the spectrum of therapeutics for treatable diseases. Therefore, this novel composition can broadly cover different types of coughs and clinically exert a better synergistic effect on coughs. Moreover, menthol and eucalyptus oil are both made from natural herbal medicines and the preparations do not contain alcohol and will be directly administered on the mucosa of the respiratory tract by aerosol inhalation. Therefore, the novel menthol-eucalyptus oil spray only requires a low dosage to produce a rapid antitussive effect and is highly safe and suitable for use across all age groups, including children. Presently, there are no cough suppressants in a form of spray on the market, and particularly, it has not been seen that both menthol and eucalyptus oil (or eucalyptol) are used as major ingredients in a preparation on the market yet.

The novel menthol-eucalyptus oil antitussive spray is an oil-in-water emulsion, comprising an oil phase, an emulsifier, and a water phase. Menthol and eucalyptus oil are dissolved in a vegetable oil first, the emulsifier and water are added in proportions, and a complete emulsification can be made through emulsification equipment. Finally, the emulsion can be diluted with water to make a spray with the required concentration. This process does not require ethanol as a co-dissolvent, so the final product is 100% ethanol-free.

The oil phase includes at least one selected from but not limited to olive oil, soybean oil, corn oil, avocado oil, sunflower seed oil, peanut oil, camellia oil, sesame oil, and refined rapeseed (or canola) oil.

The emulsifier includes at least one selected from but not limited to lecithin (soybean lecithin and/or other types of lecithin), phospholipid, gelatin, gum Arabic, tragacanth, sorbitan fatty acid, polysorbate, Pluronic F-68, polyoxyethylene fatty acid ester, polyoxyethylene fatty acid alcohol ether, and a polyoxyethylene-polyoxypropylene copolymer.

A co-emulsifier includes at least one selected from but not limited to Tween (such as Tween-80, Tween-60, Tween-40, Tween-20), methylcellulose, sodium carboxymethylcellulose, hydroxypropylcellulose, sodium alginate, tragacanth, gum Arabic, cetyl alcohol, glyceryl monostearate, stearic acid, stearyl alcohol, cholic acid, cholate, oleic acid, and oleate.

An antioxidant includes at least one selected from but not limited to vitamin E, sulfite, pyrosulfite, sodium thiosulfate, methionine, glutathione, ascorbic acid, ascorbyl palmitate, thiourea, propyl gallate, butylated hydroxyanisole (BHA), and butylated hydroxytoluene (BHT).

A preservative includes at least one selected from but not limited to sorbic acid and salts thereof (such as potassium sorbate), benzoic acid and salts thereof (such as sodium benzoate), dehydroacetic acid and sodium salts thereof, and parabens.

A flavoring agent includes at least one selected from but not limited to sugar (such as sucrose, glucose, fructose, isomerized sugar, and maltitol), non-sugar sweetener (such as saccharin sodium, sodium cyclamate, acesulfame potassium, and aspartame), natural sweetener (such as *Glycyrrhiza uralensis, Stevia rebaudiana,* and *Siraitia grosvenorii),* and fruit flavoring agents.

In the present disclosure, the eucalyptus oil can be replaced by eucalyptol (or 1,8-cineol) in a preparation to achieve a similar pharmaceutical effect.

The pharmaceutical composition does not exclude the addition of other antitussive and expectorant drugs or Chinese herbal medicines to further improve the therapeutic effect. These additional ingredients include, but are not limited to, dextromethorphan, carbocisteine, ambroxol, bromhexine, loquat, *Rhizoma Pinelliae, Fritillaria, Radix Platycodi,* snow pear, *Radix Ophiopogonis, Bulbus Lilii,* almond, *Fructus Trichosanthis, Cortex Mori, Rhizoma Anemarrhenae,* and *Radix Stemonae;* and the crude extracts, components, or compounds with antitussive and expectorant effects from traditional Chinese medicines.

In first aspect, the present disclosure provides a novel menthol-eucalyptus oil antitussive spray, including menthol and a second active ingredient selected from eucalyptus oil or eucalyptol.

Further, in the novel menthol-eucalyptus oil antitussive spray, the concentration of menthol is 0.01% to 20% in the form of weight-to-volume (w/v) and the concentration of eucalyptus oil (or equivalent eucalyptol) is 0.01% to 20% in the form of volume-to-volume (v/v).

Further, the novel menthol-eucalyptus oil antitussive spray includes but is not limited to 0.25% to 1% of menthol and 0.05 to 0.15% of eucalyptus oil (or equivalent eucalyptol); or includes but is not limited to 1% to 3% of menthol and 2.5% to 5% of eucalyptus oil (or equivalent eucalyptol), preferably 2% of menthol and 3.6% of eucalyptus oil (or equivalent eucalyptol).

Further, the novel menthol-eucalyptus oil antitussive spray is an oil-in-water emulsion, further including: an oil phase in which the oil is at least one selected from but not limited to olive oil, soybean oil, corn oil, avocado oil, tea seed or camellia oil, sunflower seed oil, peanut oil, sesame oil, refined rapeseed (or canola) oil; and an emulsifier being at least one selected from but not limited to lecithin, phospholipid, gelatin, gum arabic, tragacanth, sorbitan fatty acid, polysorbate, Pluronic F-68, polyoxyethylene fatty acid ester, polyoxyethylene fatty acid alcohol ether, polyoxyethylene-polyoxypropylene copolymer.

Further, the novel menthol-eucalyptus oil antitussive spray includes at least one selected from but not limited to co-emulsifier, antioxidant, preservative, and flavoring agent. The co-emulsifier is at least one selected from the group of compounds: Tween (such as Tween-80, Tween-60, Tween-40, Tween-20), methylcellulose, sodium carboxymethylcellulose, hydroxypropylcellulose, sodium alginate, tragacanth, gum Arabic, cetyl alcohol, glyceryl monostearate, stearic acid, stearyl alcohol, cholic acid, cholate, oleic acid, oleate. The antioxidant is at least one selected from the group of compounds: vitamin E, sulfite, pyrosulfite, sodium thiosulfate, methionine, glutathione, ascorbic acid, ascorbyl palmitate, thiourea, propyl gallate, BHA, BHT The preservative is at least one selected from the group of compounds: sorbic acid and a salt thereof (such as potassium sorbate), benzoic acid and a salt thereof (such as sodium benzoate). The flavoring agent is at least one selected from the group of components: sugar (such as sucrose, glucose, fructose, and isomerized sugar, maltitol), non-sugar sweetener (such as saccharin sodium, sodium cyclamate, acesulfame potassium, and aspartame), natural sweet plant (such as *Glycyrrhiza uralensis, Stevia rebaudiana,* and *Siraitia grosvenorii),* and fruit flavoring agent.

Further, the novel menthol-eucalyptus oil antitussive spray further includes at least one selected from but not limited to one or more of other antitussive and expectorant drugs or traditional Chinese medicines, such as dextromethorphan, carbocisteine, ambroxol, bromhexine, loquat, *Rhizoma Pinelliae, Fritillaria, Radix Platycodi,* snow pear, *Radix Ophiopogonis, Bulbus Lilii,* almond, *Fructus Trichosanthis, Cortex Mori, Rhizoma Anemarrhenae, Radix Stemonae,* and the components with antitussive and expectorant effects from traditional Chinese medicines.

In second aspect, the present disclosure provides applications of the novel menthol-eucalyptus oil antitussive spray in the production of a drug or a preparation for treating multiple types of coughs, asthma, upper respiratory tract infection, pharyngitis, and rhinitis; or in the production of an oral hygiene preparation.

In third aspect, the present disclosure provides applications of each of menthol, eucalyptus oil, and eucalyptol (or 1,8-cineol) as a principal active ingredient in the production of a drug or a preparation for treating various types of coughs, asthma, upper respiratory tract infection, pharyngitis, and rhinitis; and in the production of an oral hygiene preparation.

In fourth aspect, the present disclosure provides applications of eucalyptus oil or eucalyptol (or 1,8-cineol) in combination with menthol in the production of a drug or a preparation for treating various types of coughs, asthma, upper respiratory tract infection, pharyngitis, and rhinitis, in the preparations of which an additional active ingredient other than eucalyptol and menthol is included.

Further, the additional active ingredient other than eucalyptol and menthol in the preparation includes at least one selected from but not limited to α-thujene, α-pinene, camphene, pinene, p-cymene, ocimene, isoterpinene, α-phellandrene, α-terpinene, limonene, caryophyllene, citronellal, nerolidol, linalool, trans-pinocarveol, pine eucalyptol, and α-eudesmol.

Further, the cough is referred to inflammatory coughs caused by pathogen infections.

Further, the spray has a clear antitussive effect, and directly exerts the antitussive effect through a non-traditional, topical administration route of the respiratory tract, and has the characteristics and advantages of low dosage, rapid action, low toxicity, and fewer side effects.

### BRIEF DESCRIPTION OF THE FIGURES

FIG. 1: The experimental workflow chart of Animal Experiment 1;
FIG. 2: Effect of menthol, eucalyptus oil and their combinations on the latency and frequency of coughs after administration of sodium citrate for cough induction in Animal Experiment 1 (##, P<0.01 vs control group; **, P<0.01 vs solvent group; $$, P<0.01);
FIG. 3: The experimental workflow chart of Animal Experiment 2;
FIG. 4: Effect of menthol, eucalyptus oil and their combinations on the latency and frequency of coughs after administration of LPS followed by sodium citrate for cough induction in Animal Experiment 2 (##, P<0.01 vs control group; *, P<0.05, **, P<0.01 vs model group);
FIG.: 5 Effect of menthol, eucalyptus oil, and their combinations on the frequency of coughs after administration of LPS followed by sodium citrate for cough induction in Animal Experiment 2 ($$, P<0.01 vs control group; +, P<0.05, ++, P<0.01).

### DETAILED DESCRIPTION OF THE EMBODIMENTS

The present disclosure is further demonstrated below with specific examples. The examples are for illustration only. The protection scope of the present disclosure is defined by the claims and not limited to the following examples.

### Example 1 A novel menthol-eucalyptus oil antitussive spray

[Formula]: In this example, the novel menthol-eucalyptus oil antitussive spray includes the following components: 2 g of menthol, 4 mL of eucalyptus oil, 10 mL of olive oil, 5 g of soybean lecithin, and 20 mL of distilled water.

[Preparation method]: 2 g of menthol and 4 mL of eucalyptus oil are dissolved in 10 mL of olive oil to prepare an oil phase, 5 g of soybean lecithin and 20 mL of distilled water are added into the oil phase, and then the resulting mixture is fully emulsified in emulsifying equipment to make an oil-in-water emulsion. Finally, distilled water and the oil-in-water emulsion are mixed sufficiently, and a spray composed of 2% menthol (g/v) and 4% eucalyptus oil (v/v) or a spray with other concentrations can be prepared.

### Example 2 A novel menthol-eucalyptus oil antitussive spray

[Formula]: In this example, the novel menthol-eucalyptus oil antitussive spray includes the following components: 4 g of menthol, 2 mL of eucalyptus oil, 10 mL of refined soybean oil, 5 g of soybean lecithin, and 20 mL of distilled water.

[Preparation method]: 4 g of menthol and 2 mL of eucalyptus oil are dissolved in 10 mL of refined soybean oil to prepare an oil phase, 5 g of soybean lecithin and 20 mL of distilled water are added into the oil phase, and then the resulting mixture is fully emulsified in emulsion equipment to make an oil-in-water emulsion. Finally, distilled water and the oil-in-water emulsion are mixed sufficiently, and a spray composed of 4% menthol (g/v) and 2% eucalyptus oil (v/v) or a spray with other concentrations can be prepared.

### Example 3 A novel menthol-eucalyptus oil antitussive spray

[Formula]: In this example, the novel menthol-eucalyptus oil antitussive spray includes the following components: 4 g of menthol, 1 mL of eucalyptus oil, 10 mL of refined corn oil, 5 g of soybean lecithin, 1.2 g of vitamin E, and 20 mL of distilled water.

[Preparation method]: 4 g of menthol, 1 mL of eucalyptus oil, and 1.2 g of vitamin E are dissolved in 10 mL of refined corn oil to prepare an oil phase, 5 g of soybean lecithin and 20 mL of distilled water are added into the oil phase, and then the resulting mixture is fully emulsified in emulsifying equipment to make an oil-in-water emulsion. Finally, distilled water and the oil-in-water emulsion are mixed sufficiently, and a spray composed of 4% menthol (g/v) and 1% eucalyptus oil (v/v) or a spray with other concentrations can be prepared.

### Example 4 A novel menthol-eucalyptus oil antitussive spray

[Formula]: In this example, the novel menthol-eucalyptus oil antitussive spray includes the following components: 10 g of menthol, 2 mL of eucalyptus oil, 30 mL of refined soybean oil, 10 g of egg yolk lecithin, 0.75 g of ascorbic acid, and 20 mL of distilled water.

[Preparation method]: 10 g of menthol and 2 mL of eucalyptus oil are dissolved in 30 mL of refined soybean oil to prepare the oil phase, 10 g of egg yolk lecithin, 0.75 g of ascorbic acid, and 20 mL of distilled water are added into the oil phase, and then the resulting mixture is fully emulsified in emulsion equipment to make an oil-in-water sub-microemulsion. Finally, distilled water and the oil-in-water sub-microemulsion are mixed sufficiently, and a spray composed of 2.5% menthol (g/v) and 0.5% eucalyptus oil (v/v) or a spray with other concentrations can be prepared.

### Example 5 A novel menthol-eucalyptus oil antitussive spray

[Formula]: In this example, the novel menthol-eucalyptus oil antitussive spray includes the following components: 10 g of menthol, 1 mL of eucalyptus oil, 30 mL of olive oil, 10 g of soybean lecithin, 1 g of Tween-80, and 20 mL of distilled water.

[Preparation method]: 10 g of menthol and 1 mL of eucalyptus oil are dissolved in 30 mL of olive oil to prepare an oil phase, 10 g of soybean lecithin, 1 g of Tween-80, and 20 mL of distilled water are added into the oil phase, and then the resulting mixture is fully emulsified in emulsifying equipment to make an oil-in-water sub-microemulsion. Finally, distilled water and the oil-in-water sub-microemulsion are mixed sufficiently, and a spray composed of 2% menthol (g/v) and 0.2% eucalyptus oil (v/v) or a spray with other concentrations can be prepared.

### Example 6 A novel menthol-eucalyptus oil antitussive spray

[Formula]: In this example, the novel menthol-eucalyptus oil antitussive spray includes the following components: 8 g of menthol, 2 mL of eucalyptus oil, 30 mL of refined corn oil, 8 g of soybean lecithin, 0.5 g of Tween-80, 1 g of vitamin E, and 20 mL of distilled water.

[Preparation method]: 8 g of menthol and 2 mL of eucalyptus oil are dissolved in 30 mL of refined corn oil to prepare an oil phase, 8 g of soybean lecithin, 0.5 g of Tween-80, 1 g of vitamin E, and 20 mL of distilled water are added into the oil phase, and then the resulting mixture is fully emulsified in emulsifying equipment to make an oil-in-water sub-microemulsion. Finally, distilled water and the oil-in-water sub-microemulsion are mixed sufficiently, and a spray composed of 4% menthol (g/v) and 1% eucalyptus oil (v/v) or a spray with other concentrations can be prepared.

### Example 7 A novel menthol-eucalyptus oil antitussive spray

[Formula]: In this example, the novel menthol-eucalyptus oil antitussive spray includes the following components: 1 g of menthol, 2 mL of eucalyptus oil, 4 mL of sunflower seed oil, 1 g of Tween-60, 1 g of tragacanth, and 40 mL of distilled water.

[Preparation method]: 1 g of menthol and 2 mL of eucalyptus oil are dissolved in 4 mL of sunflower seed oil to prepare an oil phase, 1 g of Tween-60, 1 g of tragacanth, and 40 mL of distilled water are added into the oil phase, and then the resulting mixture is fully emulsified in emulsifying equipment to make an oil-in-water sub-microemulsion. Finally, physiological saline is mixed with the sub-microemulsion to prepare a spray composed of 1% menthol (g/v) and 2% eucalyptus oil (v/v) or other concentrations.

### Example 8 A novel menthol-eucalyptus oil antitussive spray

[Formula]: In this example, the novel menthol-eucalyptus oil antitussive spray includes the following components: 10 g of menthol, 2 mL of eucalyptus oil, 40 mL of refined rapeseed (or canola) oil, 2 g of sodium alginate, 10 g of polyoxyethylene fatty acid ester, and 50 mL of distilled water.

[Preparation method]: 10 g of menthol and 2 mL of eucalyptus oil are dissolved in 40 mL of refined rapeseed (canola) oil to prepare an oil phase, 2 g of sodium alginate, 10 g of polyoxyethylene fatty acid ester, and 50 mL of distilled water are added into the oil phase, and then the resulting mixture is fully emulsified in emulsifying equipment to make an oil-in-water sub-microemulsion. Finally, physiological saline is mixed with the sub-microemulsion to prepare a spray composed of 2.5% menthol (g/v) and 0.5% eucalyptus oil (v/v) or other concentrations.

### Example 9 A novel menthol-eucalyptus oil antitussive spray

[Formula]: In this example, the novel menthol-eucalyptus oil antitussive spray includes the following components: 50 g of menthol, 30 mL of eucalyptus oil, 120 mL of refined soybean oil, 40 g of polysorbate, 0.1 g of methylcellulose, and 100 mL of distilled water.

[Preparation method]: Menthol and eucalyptus oil are dissolved in the oil phase, the emulsifier, the co-emulsifier, and distilled water are added, and then the resulting mixture is fully emulsified in emulsifying equipment to make an oil-in-water microemulsion. Finally, physiological saline is mixed with the microemulsion to prepare a spray composed of 5% menthol (g/v) and 3% eucalyptus oil (v/v) or different concentrations.

### Example 10 A novel menthol-eucalyptus oil antitussive spray

[Formula]: In this example, the novel menthol-eucalyptus oil antitussive spray includes the following components: 80 g of menthol, 10 mL of eucalyptus oil, 240 mL of refined tea seed oil, 80 g of gelatin, 10 g of sodium alginate, 4 g of vitamin E, and 180 mL of distilled water.

[Preparation method]: Menthol and eucalyptus oil are dissolved in the oil phase, the emulsifier, the co-emulsifier, the antioxidant, and distilled water are added, and then the resulting mixture is fully emulsified in emulsifying equipment to make an oil-in-water emulsion. Finally, phosphate-buffered saline (PBS) is mixed with the emulsion to prepare a spray composed of 4% menthol (g/v) and 0.5% eucalyptus oil (v/v) or different concentrations.

### Example 11 A novel menthol-eucalyptus oil antitussive spray

[Formula]: In this example, the novel menthol-eucalyptus oil antitussive spray includes the following components: 3 g of menthol, 1 mL of eucalyptus oil, 9 mL of refined sesame oil, 2 g of tragacanth, 0.1 g of Tween, 0.5 g of ascorbic acid, and 5 mL of distilled water.

[Preparation method]: Menthol and eucalyptus oil are dissolved in the oil phase, the emulsifier, the co-emulsifier, the antioxidant, and distilled water are added, and then the resulting mixture is fully emulsified in emulsifying equipment to make an oil-in-water sub-microemulsion. Finally, physiological saline is mixed with the sub-microemulsion to prepare a spray composed of 1.5% menthol (g/v) and 0.5% eucalyptus oil (v/v) or different concentrations.

### Example 12 A novel menthol-eucalyptus oil antitussive spray

[Formula]: In this example, the novel menthol-eucalyptus oil antitussive spray includes the following components: 100 g of menthol, 5 mL of eucalyptus oil, 300 mL of avocado oil, 100 g of soybean lecithin, 2 g of oleic acid, 1 g of propyl gallate, and 20 mL of distilled water.

[Preparation method]: Menthol and eucalyptus oil are dissolved in the oil phase, the emulsifier, the co-emulsifier, the antioxidant, and distilled water are added, and then the resulting mixture is fully emulsified in emulsifying equipment to make an oil-in-water microemulsion. Finally, water is mixed with the microemulsion to prepare a spray composed of 5% menthol (g/v) and 0.25% eucalyptus oil (v/v) or different concentrations.

### Example 13 A novel menthol-eucalyptus oil antitussive spray

[Formula]: In this example, the novel menthol-eucalyptus oil antitussive spray includes the following components: 5 g of menthol, 2.5 mL of eucalyptus oil, 15 mL of avocado oil, 5 g of egg yolk lecithin, 1 g of gum Arabic, 1 g of vitamin E, 0.5 g of potassium sorbate, 5 g of sucrose, appropriate amount of fruit flavoring agent, and 100 mL of distilled water.

[Preparation method]: Menthol and eucalyptus oil are dissolved in the oil phase, the emulsifier, the co-emulsifier, the antioxidant, the preservative, sucrose, the fruit flavoring agent, and distilled water are added, and then the resulting mixture is fully emulsified in emulsifying equipment to make an oil-in-water sub-microemulsion. Finally, water is mixed with the sub-microemulsion to prepare a spray composed of 5% menthol (g/v) and 2.5% eucalyptus oil (v/v) or different concentrations.

### Example 14 A novel menthol-eucalyptus oil antitussive spray

[Formula]: In this example, the novel menthol-eucalyptus oil antitussive spray includes the following components: 60 g of menthol, 15 mL of eucalyptus oil, 180 mL of avocado oil, 60 g of lecithin, 5 g of Tween-80, 10 g of glutathione, 6 g of sodium benzoate, 0.5 g of aspartame, 0.5 g of fruit flavoring agent, and 120 mL of distilled water.

[Preparation method]: Menthol and eucalyptus oil are dissolved in the oil phase, the emulsifier, the co-emulsifier, the antioxidant, the preservative, the sweetener, the fruit flavoring agent, and distilled water are added, and then the resulting mixture is fully emulsified in emulsifying equipment to make an oil-in-water emulsion. Finally, water is mixed with the emulsion to prepare a spray composed of 4% menthol (g/v) and 1% eucalyptus oil (v/v) or different concentrations.

### Example 15 A novel menthol-eucalyptus oil antitussive spray

[Formula]: In this example, the novel menthol-eucalyptus oil antitussive spray includes the following components: 5 g of menthol, 6 mL of eucalyptol (also known as 1,8-Cineol), 15 mL of avocado oil, 5 g of soybean lecithin, 1 g of gum Arabic, 1 g of vitamin E, 0.5 g of potassium sorbate, 5 g of sucrose, appropriate amount of fruit flavoring agent, and 100 mL of distilled water.

[Preparation method]: Menthol and eucalyptol are dissolved in the oil phase, the emulsifier, the co-emulsifier, the antioxidant, the preservative, sucrose, the fruit flavoring agent, and distilled water are added, and then the resulting mixture is fully emulsified in emulsifying equipment to make an oil-in-water emulsion. Finally, water is mixed with the emulsion to prepare a spray composed of 5% menthol (g/v) and 6% eucalyptol (v/v) or different concentrations.

### Example 16 A novel menthol-eucalyptus oil antitussive spray

[Formula]: In this example, the novel menthol-eucalyptus oil antitussive spray includes the following components: 5 g of menthol, 1.5 mL of eucalyptus oil, 16 mL of avocado oil, 6 g of lecithin, 0.5 g of Tween-80, 1 g of ambroxol hydrochloride, 0.2 g of glutathione, 0.5 g of sodium benzoate, 2 g of aspartame, 0.5 g of fruit flavoring agent, and 100 mL of distilled water.

[Preparation method]: Menthol and eucalyptus oil are dissolved in the oil phase, the emulsifier, the co-emulsifier, the expectorant, the antioxidant, the preservative, the sweetener, the fruit flavoring agent, and distilled water are added, and then the resulting mixture is fully emulsified in emulsifying equipment to make an oil-in-water emulsion. Finally, water is mixed with the emulsion to prepare a spray composed of 5% menthol (g/v), 1.5% eucalyptus oil (v/v), and 1% ambroxol hydrochloride or different concentrations.

### Example 17 A novel menthol-eucalyptus oil antitussive spray

[Formula]: In this example, the novel menthol-eucalyptus oil antitussive spray includes the following components: 4 g of menthol, 5 mL of eucalyptol, 12 mL of olive oil, 4 g of soybean lecithin, 0.5 g of Tween-20, 2 g of carbocisteine, 0.5 g of vitamin E, 0.2 g of potassium sorbate, 3 g of sucrose, 0.1 g of fruit flavoring agent, and 100 mL of distilled water.

[Preparation method]: Menthol and eucalyptol are dissolved in the oil phase, the emulsifier, the co-emulsifier, the expectorant, the antioxidant, the preservative, sucrose, the fruit flavoring agent, and distilled water are added, and then the resulting mixture is fully emulsified in emulsifying equipment to make an oil-in-water emulsion. Finally, water is mixed with the emulsion to prepare a spray composed of 4% menthol (g/v) and 5% eucalyptol (v/v) or different concentrations.

### Example 18 A novel menthol-eucalyptus oil antitussive spray

[Formula]: In this example, the novel menthol-eucalyptus oil antitussive spray includes the following components: 2 g of menthol, 4 mL of eucalyptus oil, 10 mL of camellia oil, 2 g of soybean lecithin, 0.2 g of dextromethorphan hydrobromide, 0.5 g of oleic acid, 0.5 g of glutathione, and 100 mL of distilled water.

[Preparation method]: Menthol, eucalyptus oil, and dextromethorphan hydrobromide are dissolved in the oil phase, the emulsifier, the co-emulsifier, the antioxidant, and an appropriate amount of distilled water are added, and then the resulting mixture is fully emulsified in emulsifying equipment to make an oil-in-water microemulsion. Finally, water is mixed with the microemulsion to prepare a spray composed of 2% menthol (g/v), 4% eucalyptus oil (v/v), and 0.2% dextromethorphan hydrobromide or different concentrations.

### Example 19 A novel menthol-eucalyptus oil antitussive spray

[Formula]: In this example, the novel menthol-eucalyptus oil antitussive spray includes the following components: 2 g of menthol, 3 mL of eucalyptus oil, 3 mL of refined sesame oil, 2 g of soybean lecithin, 2 g of *Bulbus Fritillariae Cirrhosae* extract, 1 g of loquat leaf extract, 0.1 g of Tween, 0.2 g of ascorbic acid, 0.2 g of sodium sorbate, and 20 mL of distilled water.

[Preparation method]: Menthol and eucalyptus oil are dissolved in the oil phase, the emulsifier, the co-emulsifier, the Chinese herbal extract, the antioxidant, the preservative, and distilled water are added, and then the resulting mixture is fully emulsified in emulsion equipment to make an oil-in-water sub-microemulsion. Finally, physiological saline is mixed with the sub-microemulsion to prepare a spray composed of 2% menthol (g/v) and 3% eucalyptus oil (v/v) or different concentrations.

### Example 20 A novel menthol-eucalyptus oil antitussive spray

[Formula]: In this example, the novel menthol-eucalyptus oil antitussive spray includes the following components: 5 g of menthol, 8 mL of eucalyptus oil, 4 mL of corn oil, 6 g of soybean lecithin, 2 g of *Rhizoma Pinelliae* extract, 2 g of *Radix Platycodi* extract, 0.4 g of Tween, 0.5 g of ascorbic acid, 0.2 g of sodium benzoate, and 60 mL of distilled water.

[Preparation method]: Menthol and eucalyptus oil are dissolved in the oil phase, the emulsifier, the co-emulsifier, the Chinese herbal extract, the antioxidant, the preservative, and distilled water are added, and then the resulting mixture is fully emulsified in emulsifying equipment to make an oil-in-water sub-microemulsion. Finally, physiological saline is mixed with the sub-microemulsion to prepare a spray composed of 5% menthol (g/v) and 8% eucalyptus oil (v/v) or different concentrations.

### Animal Experiment 1: Study on Antitussive Effect of Menthol and Eucalyptus Oil

### Experimental Instruments:

(1) Water purification system, Labconco, USA;
(2) Low-temperature refrigerator, Forma Scientific, USA;
(3) Precision balance: AG245, Mettler Toledo, Sweden;
(4) TurboBoy nebulizing inhalation device, PARI, Germany;
(5) Video recording equipment.

### Experimental Reagents:

(1) Menthol: Jiangxi Xinsen Natural Plant Oil Co., Ltd., China (Lot No.: 2022-02-10);
(2) Eucalyptus oil: Jiangxi Xinsen Natural Plant Oil Co., Ltd., China (Lot No.: 2022-02-28);
(3) Soybean lecithin: Shenyang Tianfeng Biopharmaceutical Co., Ltd, China (Lot No.: SY-SO-211001);
(4) Olive oil: Jiangxi Xinsen Natural Plant Oil Co., Ltd., China (Lot No.: 2022-01-08);
(5) Codeine: Sinopharm Group, China (Lot No.: 200311);
(6) Sodium citrate: Sinopharm Group, China (Lot No.: 20210108);
(7) Other reagents were all AR grade products and made in China.

### Experimental Animals:

100 guinea pigs, 250 g to 300 g each, half-male and half-female, were purchased from Beijing Vital River Experimental Animal Technology Co., Ltd., China referred to Certificate Numbers 110011220104932418, 110011220104932542. The animals were reared at the general laboratory facility with 40% to 60% of humidity, and provided with a cycle of light: 12 h in dark and 12 h in light. The animals could freely access to water and food. All operations and experimental procedures complied with *the "Regulations on Animal Experiment Management".*

### Experimental Process

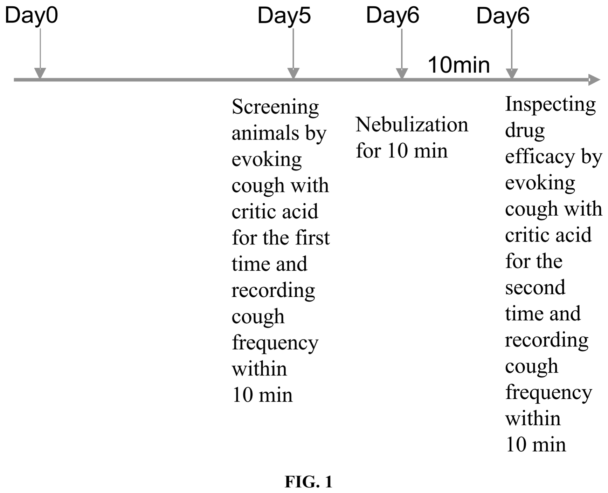

The general flowchart of experiments is shown in FIG. 1. At the initial screening step, each guinea pig was individually placed in a glass bell jar to allow adaptation for 1 min and then administered with a compressed aerosol of 0.4 M citric acid solution (instead of physiological saline for the blank control group) for 10 min. The frequency of cough was recorded during administration of the aerosol. The guinea pigs with a cough frequency of 10-30 were retained for the formal experiments later. The qualified guinea pigs were randomly divided into the corresponding groups (not fewer than 10 in each group) and were subjected to test the inhalation of the nebulized solvent, menthol, eucalyptus oil, or a combination of menthol and eucalyptus oil for 10 min. After an interval of 10 min post-inhalation of the test drug, 0.4 M citric acid solution was nebulized and given for inhalation, and the cough frequency was recorded by video from the beginning to the end of the administration of citric acid aerosol. The latency of cough is defined as the period from the inhalation of nebulized citric acid to the appearance of the first cough. The videos were recorded during the period of cough provocation in the experiment.

### Animal Groups of the Experiment:

**Table 1: Groups and administration of drugs**

| Groups | Number of animals | Cough provocation by Citric acid | Drug concentration | Route of drug administration | Time of drug administration |
|---|---|---|---|---|---|
| Model | ≥10 | | Solvent | | |
| Codeine | ≥10 | | 30 mg/mL | | |
| Menthol | ≥10 | Inhalation of 0.4 M citric acid by nebulization for 10 min | 2% | | Nebulization for 10 min before second cough provocation |
| Eucalyptus oil | ≥10 | | 3.6% | | |
| Menthol + eucalyptus oil _1 | ≥10 | | 2%+3.6% | Inhalation by nebulization | |
| Menthol + eucalyptus oil _2 | ≥10 | | 0.6%+3.6% | | |
| Menthol + eucalyptus oil _3 | ≥10 | | 2%+1.2% | | |

| | | | | | |
|---|---|---|---|---|---|
| NOTE: eucalyptus oil % (V/V), menthol % (g/V). | | | | | |

### Drug Preparation

(1) 0.4 M sodium citrate: Sodium citrate was accurately weighed, added into an appropriate volume of pure water, completely dissolved by sonication, and finally diluted to 0.4 mol/L for use later.
(2) Codeine: Codeine was accurately weighed, and ground sufficiently in a mortar, transferred into an appropriate amount of physiological saline and dissolved by sonication.
(3) Preparations of testing drugs: Initially, an oil phase was prepared by dissolving 2 g of menthol in 10 mL of olive oil (menthol group); or well mixing 3.6 mL of eucalyptus oil with 6.4 mL of olive oil (eucalyptus oil group); or adding 2 g of menthol and 3.6 mL of eucalyptus oil/0.6 g of menthol and 3.6 mL of eucalyptus oil/2 g of menthol and 1.2 mL of eucalyptus oil respectively in a corresponding volume of olive oil to make a final 10 mL solution (menthol + eucalyptus oil groups). Afterwards, 5 g of soybean lecithin (cut into small pieces beforehand) was added to 10 mL of olive oil (control group) or each of the drug groups above and dissolved by slowly stirring to form a uniform oil phase at 42°C in a water bath. Further, double distilled water was slowly added to each group in oil phase on a magnetic stirrer and fully emulsified by sufficiently stirring to mix the oil phase with water. A final emulsion was prepared in a volume of 100 mL and could be further diluted to make a working preparation with the specific concentration of drug (or drugs).

### Statistical Analysis

All experimental data were expressed as mean ± SEM, and the data-processing software SSPS16.0 was used to conduct the analysis of variance and inter-group t-test. The conclusions were made by comprehensively analyzing all data. *P*<0.05 was set up for considering statistical significance.

### Experimental Results

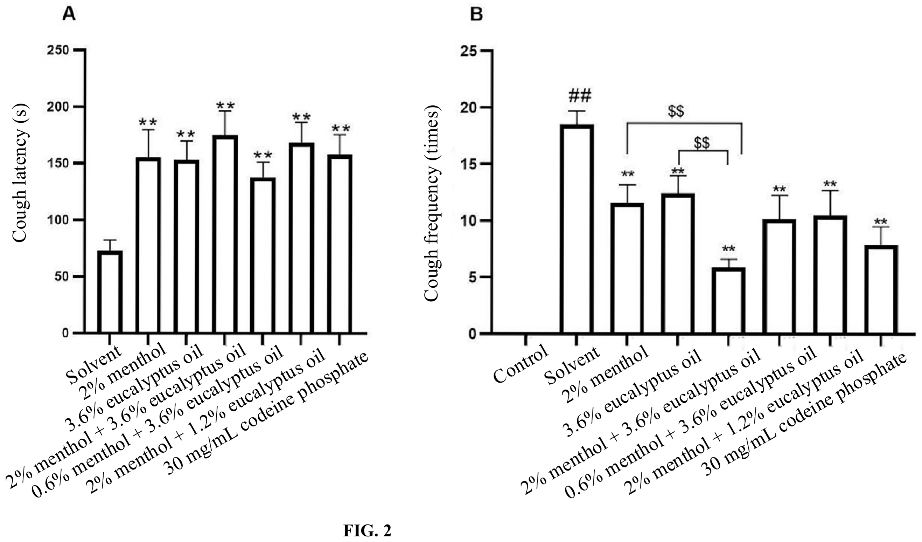

Results are shown in FIG. 2 and Table 2. Compared with the control group that only received nebulized physiological saline, 0.4 M sodium citrate could significantly evoke coughing in guinea pigs (*P*<0.01). The administration of the positive control codeine phosphate by nebulization at a dose of 30 mg/mL for 10 min significantly increased the latency and reduced the frequency of coughs when the animals were evoked by sodium citrate (*P*<0.01), indicating that the system of citric acid-evoked cough was successfully established and could be used to evaluate the antitussive efficacy of drugs. The aerosol inhalation of 2% menthol, 3.6% eucalyptus oil, or the combinations of menthol and eucalyptus oil at different concentrations significantly increased the latency and reduced the frequency of coughs in guinea pigs during the 10 min-provocation of sodium citrate (*P*<0.01). Especially, the combination of 2% menthol and 3.6% eucalyptus oil showed the best antitussive effect as significantly fewer coughs were recorded compared with the groups of menthol or eucalyptus oil alone, and it was even better than the positive control group of codeine phosphate at 30 mg/mL. The results indicate the value of the combination of menthol and eucalyptus oil for further exploration and development.

**Table 2: Effect of menthol, eucalyptus oil and their combinations on the latency and frequency of sodium citrate-evoked coughs**

| Groups | N | Cough Latency (s) | Cough Frequency (times) | Inhibition Rate of Cough (%) |
|---|---|---|---|---|
| Control | 10 | - | 0.00±0.00 | 100.00±0.00 |
| Solvent | 10 | 73.00±9.44 | 18.50±1.22^{##} | 0.00±6.61^{##} |
| 2% menthol | 12 | 155.50±20.43** | 11.58±1.72** | 37.39±9.30** |
| 3.6% eucalyptus oil | 12 | 153.25±16.68** | 12.42±1.60** | 32.88±5.06** |
| 2% menthol + 3.6% eucalyptus oil | 10 | 175.00±21.86** | 5.90±0.74** | 68.11±1.86** |
| 0.6% menthol + 3.6% eucalyptus oil | 11 | 138.00±13.18** | 10.18±2.07** | 41.96±11.42* * |
| 2% menthol +1.2% eucalyptus oil | 10 | 168.60±53.32** | 10.50±3.32** | 43.24±6.38** |
| 30 mg/mL codeine phosphate | 10 | 158.30±17.46** | 7.90±1.61** | 57.30±8.70** |

| | | | | |
|---|---|---|---|---|
| NOTE: ##, P<0.01 vs control group; **, P<0.01 vs solvent group. | | | | |

### Conclusion

2% menthol, 3.6% eucalyptus oil, and the combinations of two drugs at different concentrations significantly increased the cough latency and reduced the cough frequency in guinea pigs compared to the solvent control group. The combination of 2% menthol and 3.6% eucalyptus oil had the strongest antitussive effect, and the cough frequency was significantly fewer than that of 2% menthol or 3.6% eucalyptus oil only. The results positively indicate the value of these drugs for further exploration and development.

### Animal Experiment 2: Study on Antitussive Effect of Menthol and Eucalyptus Oil in the Model of LPS-mediated Cough Induction

### Experimental Instruments:

(1) Water purification system, Labconco, USA;
(2) Low-temperature refrigerator, Forma Scientific, USA;
(3) Precision balance: AG245, Mettler Toledo, Sweden;
(4) TurboBoy nebulizing inhalation device, PARI, Germany;
(5) Video recording equipment: Logitech camera (C302);

### Experimental Reagents:

(1) Menthol: Jiangxi Xinsen Natural Plant Oil Co., Ltd., China (Lot No.: 2022-02-10);
(2) Eucalyptus oil: Jiangxi Xinsen Natural Plant Oil Co., Ltd., China (Lot No.: 2022-02-28);
(3) Soybean lecithin: Shenyang Tianfeng Biopharmaceutical Co., Ltd., China (Lot No.: SY-S0-211001);
(4) Olive oil: Jiangxi Xinsen Natural Plant Oil Co., Ltd., China (Lot No.: 2022-01-08);
(5) Codeine: Sinopharm Group, China (Lot No.: 20210901);
(6) Sodium citrate: Sinopharm Group, China (Lot No.: 20210108);
(7) Lipopolysaccharide (LPS): Sigma (catalog number: L3129, Lot No.: 0000135946);
(7) Other reagents were all AR grade products and made in China.

### Experimental Animals:

160 guinea pigs, 250 g to 300 g each, half-male and half-female, certificate number 20220908Cdzz0412999409, were purchased from Xinjian Rabbit Farm, Dashiju Town, Xinchang County. The animals were reared at the general laboratory facility with 40% to 60% of humidity, and provided with a cycle of 12 h in the dark and 12 h in light. The animals could freely access to water and food. All operations and experimental procedures complied with the "Regulations on Animal Experiment Management".
Production license: SCXK (Zhejiang) 2020-0005.
Application license: SCXK (Zhejiang) 2021-0019.

### Experimental Methods:

### 1. Exploration on a novel model for studying inflammatory coughs

Currently, there are barely reports on the models of inflammatory coughs in China and other countries. To closely mimic the occurrence of actual coughs in people suffering from acute respiratory infection, a novel inflammatory cough model was explored ahead of the projected experiments. This novel model was based on the citric acid-induced cough model, but the animals were subject to a LPS pretreatment before beginning with the cough provocation of citric acid. The results showed that the pre-induction of inflammation by LPS could significantly evoke coughs in guinea pigs at a lower concentration (0.2 M) of citric acid at which the frequency of coughs was equivalent to the one in the conventional model with cough provocation at 0.4 M citric acid.

### 2. Whole Process of the Experiments

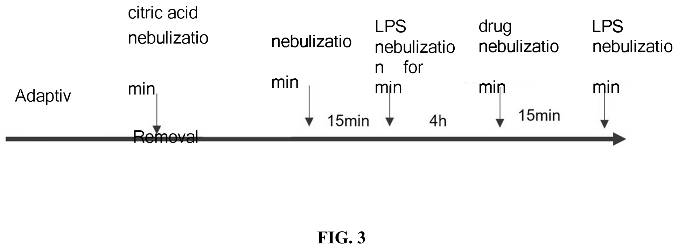

After 5 days of adaptive feeding, the animal was individually placed in a glass bell jar to allow adaptation for 1 min, and then administrated with the compressed aerosol of 0.4 M citric acid solution (instead of physiological saline for the blank control group) for 10 min. The frequency of cough was recorded during administration of the citric acid aerosol. The guinea pigs with a cough frequency of 10-30 were retained for the formal experiments later. The qualified guinea pigs were randomly divided into the corresponding groups (not fewer than 8 in each group) and were subjected to test the inhalation of the nebulized solvent, menthol, eucalyptus oil, or a combination of menthol and eucalyptus oil for 10 min. After an interval of 15 min post inhalation of the test drug, the animals were administered with the aerosol of 1 mg/mL LPS. 4 hours post the LPS nebulization, the animals were subjected to the second inhalation of the nebulized solvent, menthol, eucalyptus oil, or the combination of menthol and eucalyptus oil for 10 min. Fifteen minutes later, the aerosol of 0.2 M citric acid was administered for cough induction, and the process was recorded by video during the inhalation of citric acid. The duration from the inhalation of the nebulized citric acid to the emergence of first cough was designated as the latency. The control group used solvent for nebulization and cough evoking. The whole flowchart of experiment was shown in FIG. 3.

### 3. Animal Groups of the Experiment:

**Table 3: Groups and administration of drugs**

| Groups | N | Cough | Drug concentration | Route of drug | | Time of drug |
|---|---|---|---|---|---|---|
| | | provocation by citric acid | | | administration | administration |
| Blank control (physiological saline) | 6 | / | Olive oil | | / | / |
| Model | 9 | Inhalation of 0.2 M citric acid + 1 mg/mL LPS by nebulization for 10 min | Olive oil | | Inhalation by nebulization for 10 min | Nebulization for 10 min with eucalyptus oil and/or menthol 15 min before nebulization of LPS and citric acid |
| Codeine | 9 | | 30 mg/mL | | | |
| 0.06% menthol | 8 | | 0.06% menthol | - | | |
| 0.25% menthol | 8 | | 0.25% menthol | - | | |
| 1.0% menthol | 8 | | 1.0% menthol | - | | |
| 4.0% menthol | 8 | | 4.0% menthol | - | | |
| 0.05% eucalyptus oil | 8 | | - | 0.05% eucalyptus oil | | |
| *0.15%* eucalyptus oil | 8 | | - | 0.15% eucalyptus oil | | |
| 0.45% eucalyptus oil | 8 | | - | 0.45% eucalyptus oil | | |
| 0.06% menthol + 0.05% eucalyptus oil | 8 | | 0.06% menthol | 0.05% eucalyptus oil | | |
| 0.06% menthol + 0.15% eucalyptus oil | 8 | | 0.06% menthol | 0.15% eucalyptus oil | | |
| 0.25% menthol + 0.05% eucalyptus oil | 8 | | 0.25% menthol | 0.05% eucalyptus oil | | |
| 0.25% menthol + 0.15% eucalyptus oil | 8 | | 0.25% menthol | 0.15% eucalyptus oil | | |
| 1.0% menthol + 0.05% eucalyptus oil | 8 | | 1.0% menthol | 0.05% eucalyptus oil | | |
| 1.0% menthol + 0.15% eucalyptus oil | 8 | | 1.0% menthol | 0.15% eucalyptus oil | | |

### 4. Drug Preparation

(1) 0.4 M citric acid: Citric acid was accurately weighed, added into an appropriate volume of pure water to make a final concentration of 0.4 M, and completely dissolved by sonication.
(2) 0.2 M citric acid: Citric acid was accurately weighed, added into an appropriate volume of pure water to make a final concentration of 0.2 M, and completely dissolved by sonication.
(3) Codeine: Codeine was accurately weighed, placed in a mortar and ground repeatedly, added into an appropriate amount of physiological saline and completely dissolved by sonication.
(4) LPS preparation: LPS was accurately weighed, added into an appropriate amount of physiological saline to make a final concentration of 1 mg/mL, and completely dissolved by vortexing.
(5) Preparations of testing drugs: Initially, 4 g of menthol was dissolved in 10 mL of olive oil (menthol group), or 0.45 mL of eucalyptus oil was sufficiently mixed with 9.55 mL of olive oil (eucalyptus oil group). Afterwards, 5 g of soybean lecithin (previously cut into small pieces) was added to 10 mL of olive oil (control group) or each of the drug groups above, and dissolved by slowly stirring to form a uniform oil phase at 42°C in a water bath. Further, double distilled water was slowly added to each group in oil phase on a magnetic stirrer and fully emulsified by sufficiently stirring to mix the oil phase with water. A final emulsion was prepared in a volume of 100 mL, and could be further diluted to make a working preparation with the specific concentration of drug. Different amount of menthol and eucalyptus oil were co-dissolved in olive oil to make the various combinations of menthol and eucalyptus oil.

### 5. Statistical Analysis

All experimental data were expressed as Mean ± SEM and analyzed by single factor method using the data-processing software SSPS16.0, and the conclusions were made by comprehensively analyzing all data. Analysis of synergism was conducted by the factorial interaction and main effect using the software SPSS16.0. P<0.05 was set up for considering statistical significance.

### Experimental Results

### (I) Antitussive effect of menthol and eucalyptus oil in the novel model by the combination of LPS pretreatment with citric acid cough induction

In this model, guinea pigs were administered with 1 mg/mL LPS by nebulization and 4 hours later given with 0.2 M citric acid for 10 min to evoke coughs. The frequency and latency of coughs were recorded within 10 min. The results showed that 0.2 M citric acid could significantly induce coughs compared with the blank control group (P<0.01). Meanwhile, the administrations of 30 mg/mL codeine before inhalations of LPS and citric acid could significantly reduce the frequency of coughs but increase the latency of coughs in the codeine positive control group (P<0.01), indicating that the LPS-citric acid system was valid for cough provocation and could be used to evaluate the efficacy of antitussive drugs.

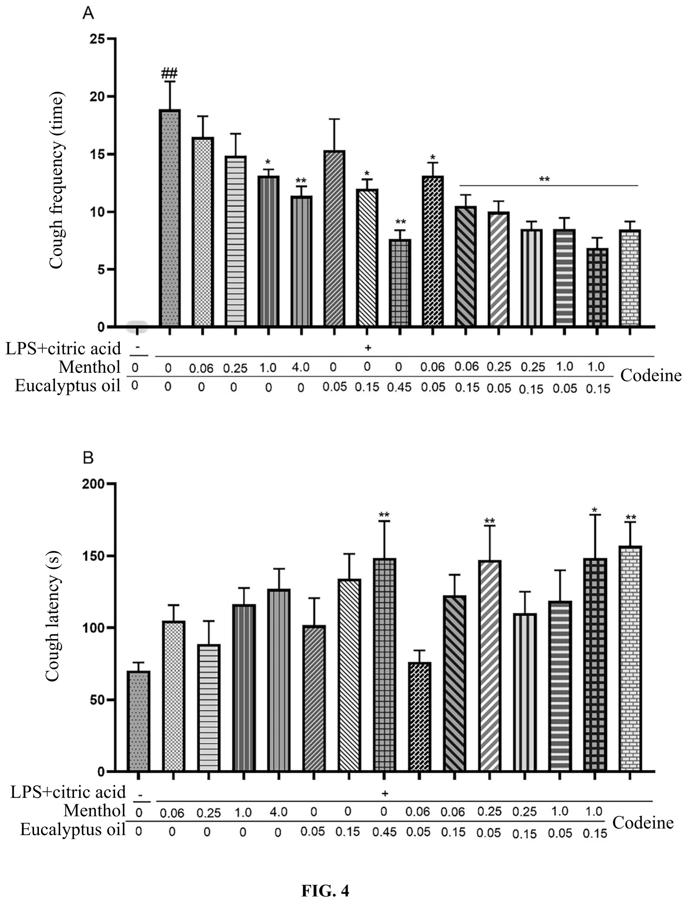

To test antitussive effect, menthol, eucalyptus oil, or the combinations of menthol and eucalyptus oil were respectively administered before the inhalations of LPS and 0.2 M citric acid by nebulization in the different groups of animals. The results showed that 0.06%, 0.25%, 1.0%, and 4% of menthol could all reduce the frequency of coughs in a dose-dependent manner, and statistical significance was specifically demonstrated in the groups of 1.0% and 4% of menthol (P<0.01-0.05). Similarly, 0.05%, 0.15%, and 0.45% of eucalyptus oil could all reduce the frequency of coughs in a dose-dependent manner, and statistical significance was specifically demonstrated in the groups of 0.15% and 0.45% of eucalyptus oil (*P<*0.01-0.05). Furthermore, all the combinations of menthol (0.06%, 0.25%, and 1.0%) and eucalyptus oil (0.05% and 0.15%) could significantly inhibit the induction of coughs in a dose-dependent manner in the LPS-citric acid model (P<0.01-0.05) (Panel A of FIG. 4). Although the latency of cough was increased by a dose-dependent manner in each group of the test drugs, a higher deviation was seen within each group. Statistically, the significance was only shown in the groups of 0.45% eucalyptus oil, 0.25% menthol + 0.15% eucalyptus oil, and 1.0% menthol + 0.15% eucalyptus oil compared with the model group (P<0.01-0.05) (Panel B of FIG. 4).

**Table 4: Antitussive effect of menthol, eucalyptus oil and their combinations in the model of cough induction using LPS and citric acid (Mean±SEM)**

| Groups | N | Cough Latency (s) | Cough Frequency | Cough Frequency Suppression Rate (%) | |
|---|---|---|---|---|---|
| | | | | Actual Value | Expected Value of Additive Effect from |
| | | | | | Two Drugs☆ |
| Control | 6 | - | 0.00±0.00 | 100.00±0.00 | - |
| Model | 9 | 80.22±8.46 | 18.89±2.43^{##} | 0.00±12.86^{##} | - |
| Codeine | 9 | 134.78±9.72** | 8.44±0.71** | 55.29±3.86** | - |
| 0.06% menthol | 8 | 104.88±10.85 | 16.50±1.78 | 12.65±9.44 | - |
| 0.25% menthol | 8 | 88.75±15.96 | 14.88±1.90 | 21.25±10.08 | - |
| 1.0% menthol | 8 | 116.38±11.30 | 13.13+0.55* | 28.53±3.00* | - |
| 4.0% menthol | 8 | 128.13±13.84 | 11.38±0.82** | 39.78±4.35** | - |
| 0.05% eucalyptus oil | 8 | 101.75±18.89 | 15.38±2.67 | 18.60±14.15 | - |
| 0.15% eucalyptus oil | 8 | 134.13±17.19 | 12.00±0.82* | 36.47±4.36* | - |
| 0.45% eucalyptus oil | 8 | 148.63±25.49** | 7.63±0.78** | 59.63±4.12** | - |
| 0.06% menthol + 0.05% eucalyptus oil | 8 | 76.38±8.00 | 13.13±1.14* | 30.51±6.04* | 31.25 |
| 0.06% menthol + 0.15% eucalyptus oil | 8 | 122.50±14.33 | 10.50±0.96** | 44.41+5.10** | 49.12 |
| 0.25% menthol + 0.05% eucalyptus oil | 8 | 147.25±23.68** | 10.00±0.93** | 47.06±4.90** | **39.85** |
| 0.25% menthol + 0.15% eucalyptus oil | 8 | 110.25±14.87 | 8.50±0.65** | 55.00±3.47** | 57.72 |
| 1.0% menthol + 0.05% eucalyptus oil | 8 | 118.75±21.17 | 8.50±0.96** | 55.00±5.10** | **47.13** |
| 1.0% menthol + 0.15% eucalyptus oil | 8 | 148.38±30.15* | 6.88±0.88** | 67.57±3.53** | **65** |

| | | | | | |
|---|---|---|---|---|---|
| ☆ Representing the sum of cough suppression rates of individual menthol and individual eucalyptus oil. | | | | | |

### (II) Synergistic analysis for the combinations of menthol and eucalyptus oil

Based on the above analysis, it was found that menthol, eucalyptus oil, and the combinations of two drugs could effectively suppress the cough induction by the co-treatment of LPS and citric acid in guinea pigs. To further analyze whether the combinations of menthol and eucalyptus oil could result in the synergism of antitussive effects, factorial analysis were conducted for the interaction effect using the data from the single drug groups and the two drug groups.

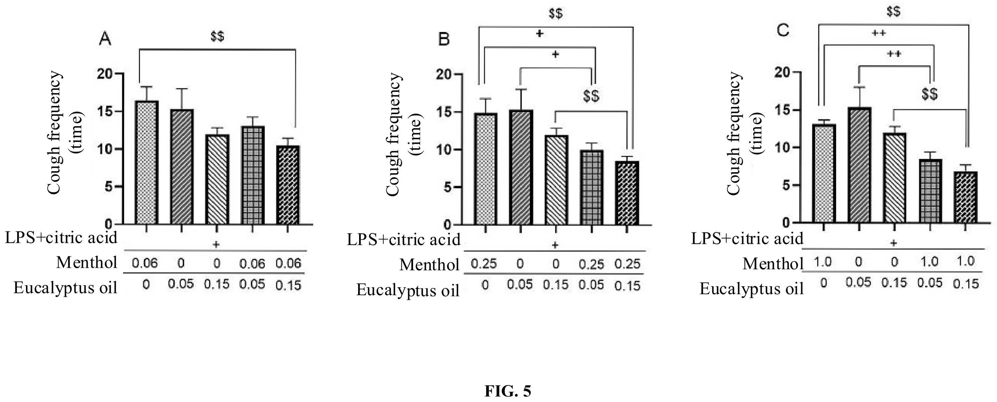

The results showed that the combination of 0.06% menthol with 0.15% eucalyptus oil was significantly better than the single use of 0.06% menthol on the reduction of cough frequency (Panel A of FIG. 5, Table 5), and the combinations of menthol at 0.25% or 1.0% with eucalyptus oil at 0.05% or 0.15% were even more significantly better than the single uses of menthol or eucalyptus oil on the reduction of cough frequency (Panel C of FIG. 5, Table 5). These suggest that the combinatorial therapy showed a significant advantage over the single use of menthol or eucalyptus oil. However, the interaction in factorial analysis did not show synergistic effect on the combination of menthol and eucalyptus oil (P>0.05) though the effect of the combinations of 0.25% or 1.0% menthol with 0.05% or 0.15% eucalyptus oil were significantly better than or at least not worse than the sum of the antitussive effects obtained from the single use of two drugs (Table 4). Under the condition of this model, the results still prove that the combination of menthol and eucalyptus oil can reach a desirable antitussive effect and has a long-term prospect of development.

**Table 5: Statistics on the antitussive effect of the combinations of menthol and eucalyptus oil in the model of LPS-citric acid cough provocation**

| Groups and Dosage | 0% | 0.06% Menthol | 0.25% Menthol | 1.0% Menthol |
|---|---|---|---|---|
| 0% | - | - | - | |
| 0.05% Eucalyptus Oil | - | | $,+ | $$,++ |
| 0.15% Eucalyptus Oil | | $$ | $$,++ | $$,++ |

| | | | | |
|---|---|---|---|---|
| NOTES: *, P<0.05, **, P<0.01 vs model group; +, P<0.05, ++, P<0.01 vs the single use of eucalyptus oil group at the same dosage; $, P<0.05, $$, P<0.01 vs the single use of menthol group at the same dosage. | | | | |

### (III) Comparison of the antitussive effects of drugs between the original model and the novel model of inflammatory coughs

To better compare and interpret the experimental results from the traditional model and the novel model of inflammatory coughs, the partial data from the two experiments were summarized in Table 6. In the original model, 2% menthol and 3.6% eucalyptus oil attained the similar rates of cough suppression at 37.39% and 33.88% respectively, and the concentration ratio of two drugs was approximately 1:2. Whereas, the combination of 2% menthol and 3.6% eucalyptus oil resulted in a cough suppression rate of 68.11%, proving that the antitussive effect could be significantly enhanced when two drugs were administered together. In the novel model, 4% menthol and 0.15% eucalyptus oil fulfilled the similar rates of cough suppression at 39.78% and 36.47% respectively, and the concentration ratio of two drugs was over 20:1. It was found that the antitussive effect of menthol on inflammatory coughs was reduced while the antitussive effect of eucalyptus oil on inflammatory coughs was significantly improved. The co-administration of 1% menthol and 0.15% eucalyptus oil could obtain a cough suppression rate of 67.57%, indicating that the combination of two drugs could significantly reduce their dosages in the novel model of inflammatory coughs, especially the dosage of eucalyptus oil.

In comparison, by the similar rates of cough suppression, 2% menthol reached a rate of 37.39% in the original model while 4% menthol achieved a rate of 39.78% in the novel model. Therefore, the antitussive effect of menthol on inflammatory coughs was approximately decreased by 2 times. Since 0.2 M sodium citrate was used to evoke cough in the novel model, which was only half of the concentration used in the original model, the actual antitussive effect of menthol on inflammatory coughs was decreased by about 4 times. Conversely, 3.6% eucalyptus oil was required to achieve a cough suppression rate of 33.88% in the original model while 0.15% eucalyptus oil was required to obtain a rate of 36.47% in the novel model. Thus, the antitussive efficacy was at least increased by 24 times. For the same reason, 0.2 M sodium citrate was only used for cough induction in the novel model, which was half of the concentration in the original model, hence the actual antitussive efficacy of eucalyptus oil for inflammatory coughs was at least increased by 12 times.

Based on the results from both animal experiments, it can be concluded that the antitussive mechanisms of menthol and eucalyptus oil are different. The former showed better activities on non-inflammatory coughs; however, the latter was particularly much better on inflammatory coughs. Therefore, the preparation composed of these drugs could complement their advantages to achieve a synergistic effect in terms of mechanisms of antitussive action.

**Table 6: Comparison of experimental results between the traditional citric acid model and the novel inflammatory cough model in guinea pigs**

| Drugs | Experiment (1): Traditional Citric Acid Model | | Experiment (2): Novel LPS-Citric Acid Model | |
|---|---|---|---|---|
| | Concentration | Cough Suppression Rate (%) | Concentration | Cough Suppression Rate (%) |
| Menthol | 2% menthol | 37.39±9.30 | 0.06% menthol | 12.65±9.44 |
| | | | 0.25% menthol | 21.25±10.08 |
| | | | 1.0% menthol | 28.53±3.00 |
| | | | 4.0% menthol | **39.78±4.35** |
| Eucalyptus Oil | 3.6% eucalyptus oil | 32.88±5.06 | 0.05% eucalyptus oil | 18.60±14.15 |
| | | | 0.15% eucalyptus oil | **36.47±4.36** |
| | | | 0.45% eucalyptus oil | 59.63±4.12 |
| Menthol + Eucalyptus Oil | 0.6% menthol + 3.6% eucalyptus oil | 41.96±11.42 | 0.06% menthol + 0.05% eucalyptus oil | 30.51±6.04 |
| | | | 0.06% menthol + 0.15% eucalyptus oil | 44.41±5.10 |
| | 2% menthol ± 1.2% eucalyptus oil | 43.24+6.38 | 0.25% menthol + 0.05% eucalyptus oil | 47.06+4.90 |
| | | | 0.25% menthol + 0.15% eucalyptus oil | 55.00+3.47 |
| | 2% menthol + 3.6% eucalyptus oil | **68.11±1.86** | 1.0% menthol + 0.05% eucalyptus oil | 55.00±5.10 |
| | | | 1.0% menthol + 0.15% eucalyptus oil | **67.57±3.53** |

### Conclusion

At present, there is no dedicated model for screening antitussive drugs and studying the mechanisms of antitussive effect on inflammatory coughs in China and other countries. The pretreatment of LPS followed with cough induction by 0.2 M citric acid was evidently a new attempt to establish a novel inflammatory cough model. Preliminary model exploration confirmed that the cough frequency was significantly higher under the LPS-0.2 M citric acid provocation in guinea pigs than under the sole provocation with 0.2 M citric acid. Nebulization of 30 mg/mL codeine significantly reduced the frequency of coughs and increased the latency of coughs in guinea pig, indicating that the LPS-citric acid cough-induction system was successfully established and could be used for the exploration and evaluation of the drug antitussive efficacy on inflammatory coughs.

By applying this inflammatory cough model, it was further found that 0.06%, 0.25%, 1.0%, and 4% of menthol could reduce the frequency of coughs in a dose-dependent manner, and 1.0% and 4% of menthol statistically attained the significance in cough suppression. Similarly, 0.05%, 0.15%, and 0.45% of eucalyptus oil could reduce the frequency of coughs in a dose-dependent manner, and 0.15% and 0.45% of eucalyptus oil statistically reached the significance in cough suppression. Furthermore, the combinations of menthol at 0.25% and 1.0% and eucalyptus oil at 0.05% and 0.15% could all significantly suppress the LPS-0.2M citric acid-induced coughs, and were all significantly better than the single use of each of the drugs. In particular, when compared with original model, it was found that the efficacy of menthol on inflammatory coughs was reduced, but the efficacy of eucalyptus oil was significantly enhanced on inflammatory coughs. These findings indicate that the combination of menthol and eucalyptus oil can achieve a synergistic effect in terms of their mechanisms of antitussive action. In conclusion, the antitussive preparations composed of menthol and eucalyptus oil show evident potentials for further exploration and development.

### Cited References:

1. Banner, K. H., F. Igney, and C. Poll, TRP channels: emerging targets for respiratory disease. Pharmacol Ther, 2011. 130(3): p. 371-84.
2. Zhou, Y., et al., Sensitivity of bronchopulmonary receptors to cold and heat mediated by transient receptor potential cation channel subtypes in an ex vivo rat lung preparation. Respir Physiol Neurobiol, 2011. 177(3): p. 327-32.
3. Laude, E. A., A.H. Morice, and T.J. Grattan, The antitussive effects of menthol, camphor and cineole in conscious guinea-pigs. Pulm Pharmacol, 1994. 7(3): p. 179-84.
4. Plevkova, J., et al., The role of trigeminal nasal TRPM8-expressing afferent neurons in the antitussive effects of menthol. J Appl Physiol (1985), 2013. 115(2): p. 268-74.
5. Millqvist, E., E. Ternesten-Hasseus, and M. Bende, Inhalation of menthol reduces capsaicin cough sensitivity and influences inspiratory flows in chronic cough. Respir Med, 2013. 107(3): p. 433-8.
6. Buday, T., et al., Modulation of cough response by sensory inputs from the nose - role of trigeminal TRPA1 versus TRPM8 channels. Cough, 2012. 8(1): p. 11.
7. Wise, P.M., P.A. Breslin, and P. Dalton, Sweet taste and menthol increase cough reflex thresholds. Pulm Pharmacol Ther, 2012. 25(3): p. 236-41.
8. Paul, I. M., Therapeutic Options for Acute Cough Due to Upper Respiratory Infections in Children. Lung, 2012. 190(1): p. 41-44.
9. Paul, I. M., et al., Vapor Rub, Petrolatum, and No Treatment for Children with Nocturnal Cough and Cold Symptoms. Pediatrics, 2010. 126(6): p. 1092-1099.
10. Her, L., et al., Efficacy and Safety of Eucalyptus for Relieving Cough: A Systematic Review and Meta-Analysis of Randomized Controlled Trials. Journal of Integrative and Complementary Medicine, 2022*.*
11. Fischer, J. and U. Dethlefsen, Efficacy of cineole in patients suffering from acute bronchitis: a placebo-controlled double-blind trial. Cough, 2013. 9(1): p. 25.
12. Kardos, P., Khaletskaya, O. & Kropova, O., Efficacy and safety of Cineole (Soledum®) in the treatment of patients with acute bronchitis: results of an open-label randomized clinical phase III study. Clinical Phytoscience, 2021. Volume 7, Article number: 83.
13. Juergens, U. R., et al., Anti-inflammatory activity of 1.8-cineol (eucalyptol) in bronchial asthma: a double-blind placebo-controlled trial. Respir Med, 2003. 97(3): p. 250-6.
14. Juergens, U. R., Anti-inflammatory properties of the monoterpene 1.8-cineole: current evidence for co-medication in inflammatory airway diseases. Drug Res (Stuttg), 2014. 64(12): p. 638-46.
15. Bachir, R.G. and M. Benali, Antibacterial activity of the essential oils from the leaves of Eucalyptus globulus against Escherichia coli and Staphylococcus aureus. Asian Pac J Trop Biomed, 2012. 2(9): p. 739-42.
16. Mulyaningsih, S., et al., Antibacterial activity of essential oils from Eucalyptus and of selected components against multidrug-resistant bacterial pathogens. Pharm Biol, 2011. 49(9): p. 893-9.

## Claims

1. A novel menthol-eucalyptus oil antitussive spray, comprising active ingredients of menthol and a second active ingredient, the second active ingredient being eucalyptus oil or eucalyptol;
in the novel menthol-eucalyptus oil antitussive spray, a concentration of menthol is in a range from 0.01% to 20% with a weight-to-volume ratio (w/v) and the second active ingredient is in a concentration from 0.01% to 20% with a volume ratio (v/v); and
the novel menthol-eucalyptus oil antitussive spray is an oil-in-water emulsion, and further comprises:
an oil phase, in which includes at least one selected from but not limited to at least one selected from olive oil, soybean oil, corn oil, avocado oil, tea seed oil, sunflower seed oil, peanut oil, sesame oil, and refined rapeseed oil; and
an emulsifier, which includes at least one selected from but not limited to lecithin, phospholipid, gelatin, gum Arabic, tragacanth, sorbitan fatty acid ester, polysorbate, Pluronic F-68, polyoxyethylene fatty acid ester, polyoxyethylene fatty acid alcohol ether, and a polyoxyethylene-polyoxypropylene copolymer.

2. The novel menthol-eucalyptus oil antitussive spray as claimed in claim 1, wherein in the novel menthol-eucalyptus oil antitussive spray, the w/v concentration of the menthol is in a range of 0.25% to 1%, and the v/v concentration of the second active ingredient is in a range of 0.05% to 0.15%; alternatively,
in the novel menthol-eucalyptus oil antitussive spray, the w/v concentration of the menthol is in a range of 1% to 3%, and the v/v concentration of the second active ingredient is in a range of 2.5% to 5%.

3. The novel menthol-eucalyptus oil antitussive spray as claimed in claim 1 or 2, further comprises but is not limited to at least one of:
a co-emulsifier, wherein the co-emulsifier is at least one selected but not limited from the group consisting of Tween, methylcellulose, sodium carboxymethylcellulose, hydroxypropylcellulose, sodium alginate, tragacanth, gum Arabic, cetyl alcohol, glyceryl monostearate, stearic acid, stearyl alcohol, cholic acid, cholate, oleic acid, and oleate;
an antioxidant, wherein the antioxidant is at least one selected from but not limited to the group consisting of vitamin E, sulfite, pyrosulfite, sodium thiosulfate, methionine, glutathione, ascorbic acid, ascorbyl palmitate, thiourea, propyl gallate, tert-butyl-4-hydroxyanisole, and di-tert-butyl-p-cresol;
a preservative, wherein the preservative is at least one selected from but not limited to the group consisting of sorbic acid and a salt thereof, and benzoic acid and a salt thereof; and
a flavoring agent, wherein the flavoring agent is at least one selected from but not limited to the group consisting of sugar, maltitol, a non-sugar sweetener, and a fruit flavoring agent.

4. The novel menthol-eucalyptus oil antitussive spray as claimed in claim 3, wherein the sugar comprises at least one selected from but not limited to the group consisting of sucrose, glucose, fructose, and an isomerized sugar; or
the non-sugar sweetener comprises but is not limited to at least one selected from but not limited to the group consisting of a natural sweetener and an artificial sweetener;
the natural sweetener comprises at least one selected from but not limited to the group consisting of *Glycyrrhiza uralensis, Stevia rebaudiana,* and *Siraitia grosvenorii;* and
the artificial sweetener comprises at least one selected from but not limited to the group consisting of saccharin sodium, sodium cyclamate, and acesulfame.

5. The novel menthol-eucalyptus oil antitussive spray as claimed in claim 3, wherein the sugar comprises one selected from but not limited to the group consisting of sucrose, glucose, fructose, and an isomerized sugar;
the non-sugar sweetener comprises one selected from but not limited to the group consisting of a natural sweetener and an artificial sweetener;
the natural sweetener comprises one selected from but not limited to the group consisting of *Glycyrrhiza uralensis, Stevia rebaudiana,* and *Siraitia grosvenorii;* and
the artificial sweetener comprises one selected from but not limited to the group consisting of saccharin sodium, sodium cyclamate, and acesulfame.

6. The novel menthol-eucalyptus oil antitussive spray as claimed in claim 3, wherein the sugar is at least one selected from the group consisting of sucrose, glucose, fructose, and an isomerized sugar;
the non-sugar sweetener is at least one selected from the group consisting of a natural sweetener and an artificial sweetener;
the natural sweetener is at least one selected from the group consisting of *Glycyrrhiza uralensis, Stevia rebaudiana,* and *Siraitia grosvenorii;* and
the artificial sweetener is at least one selected from the group consisting of saccharin sodium, sodium cyclamate, acesulfame, and aspartame.

7. The novel menthol-eucalyptus oil antitussive spray as claimed in claim 3, wherein the fruit flavoring agent comprises at least one selected from the group consisting of a natural fruit flavoring agent and an artificial fruit flavoring agent;
the natural fruit flavoring agent comprises at least one selected from but not limited to the group consisting of strawberry, blueberry, blackberry, sweet orange, tangerine, grapefruit, lemon, grape, apple, ananas, passion fruit, mango, banana, lychee, longan, pear, plum, watermelon, chestnut, jujube, and hawthorn.

8. The novel menthol-eucalyptus oil antitussive spray as claimed in claim 3, further comprises but is not limited to other Chinese medicinal ingredients and Western medicinal ingredients with antitussive and/or expectorant activities, wherein the other Chinese medicinal ingredients and Western medicinal ingredients with antitussive and/or expectorant activities comprises and is not limited to at least one selected from the group consisting of: dextromethorphan, carbocisteine, ambroxol, bromhexine, loquat, *Rhizoma Pinelliae, Fritillaria, Radix Platycodi,* snow pear, *Radix Ophiopogonis, Bulbus Lilii,* almond, *Fructus Trichosanthis, Cortex Mori, Rhizoma Anemarrhenae,* and *Radix Stemonae.*

9. The use of the novel menthol-eucalyptus oil antitussive spray as claimed in any one of claims 1 to 8 in manufacture of a drug or a preparation for treating various types of coughs, asthma, upper respiratory tract infection, pharyngitis, or rhinitis.

10. The use of the novel menthol-eucalyptus oil antitussive spray as claimed in any one of claims 1 to 8 in manufacture of an oral hygiene preparation.

11. The use as claimed in claim 9, wherein the coughs comprise a cough related to inflammation caused by infections of a pathogen.

12. The use as claimed in claim 9, 10, or 11, wherein the novel menthol-eucalyptus oil antitussive spray is topically administered via respiratory tract.

13. The use of any one of menthol, eucalyptus oil, and eucalyptol as a principal active ingredient in manufacture of a drug or a preparation for treating various types of coughs, asthma, upper respiratory tract infection, pharyngitis, and rhinitis.

14. The euse of any one of menthol, eucalyptus oil, and eucalyptol as a principal active ingredient in manufacture of an oral hygiene preparation.

15. The use of eucalyptus oil or eucalyptol in combination with menthol in manufacture of a drug or a preparation for treating various types of coughs, asthma, upper respiratory tract infection, pharyngitis, and rhinitis, wherein the preparation contains or does not contain any other active ingredients than eucalyptol and menthol.

16. The use of eucalyptus oil or eucalyptol in combination with menthol in manufacture of an oral hygiene preparation, wherein the oral hygiene preparation contains or does not contain any other active ingredients than eucalyptol and menthol.
